# EUROPEAN PATENT APPLICATION

(11) **EP 1 357 108 A1**
(43) Date of publication of application: **29.10.2003**
(21) Application number: 02716419.3
(22) Date of filing: 28.01.2002
(51) Int. Cl.: C07C 257/18, C07D 211/26

(54) **BENZAMIDINE DERIVATIVES AND PROCESS FOR PRODUCTION THEREOF**

(30) Priority: 29.01.2001 JP 2001019684
(71) Applicant: TEIJIN LIMITED, Osaka-shi Osaka 541-0054 (JP)
(72) Inventor: HARA, Takayuki C/O TEIJIN LIMITED, Hino-shi, Tokyo 191 -0065 (JP); MINOSHIMA, Toru C/O TEIJIN LIMITED, Iwakuni-shi, Yamaguchi 740-0014 (JP); TABE, Masayasu C/O TEIJIN LIMITED, Tokyo 100-0011 (JP)
(74) Representative: Hallybone, Huw George
(86) International application number: JP0200607
(87) International publication number: WO02060861

(57) **Abstract**

A process for production of an amidine derivative represented by the following Formula (II) [wherein, R represents a hydrogen atom, an unsubstituted or substituted phenyl group, an unsubstituted or substituted C1-C10 alkyl group, an unsubstituted or substituted C1-C10 alkoxy group, an unsubstituted or substituted C1-C10 alkoxycarbonyl group, or a hydroxyl group present at the ortho, meta, or para position] or a salt thereof, comprising the steps of reducing an amidoxime derivative represented by the following Formula (I) [wherein, R represents a hydrogen atom, an unsubstituted or substituted phenyl group, an unsubstituted or substituted C1-C10 alkyl group, an unsubstituted or substituted C1-C10 alkoxy group, an unsubstituted or substituted C1-C10 alkoxycarbonyl group, or a hydroxyl group present at the ortho, meta, or para position] with zinc in an acetic acid as a solvent.

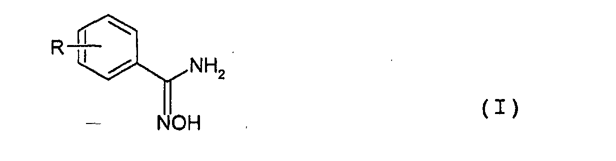

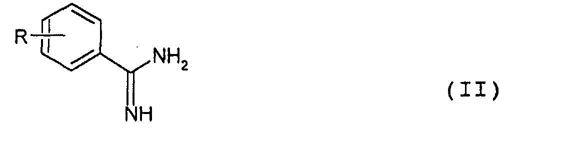

## Description

### Field of the Invention

The present invention relates to amidine derivatives and process for the production thereof. More specifically, it relates to novel amidine derivatives and process, for the production thereof, which comprises reducing the amidoximes using zinc.

### Background Art

As one of the methods for synthesizing amidine derivatives, there is known a method that employs the reduction of amidoxime derivatives (for example, Chem. Pharm. Bull., 1978, 26:1929; J. Org. Chem., 1971, 36:466; J. Chem. Soc., Chem. Commun., 1975, 761; J. Med. Pharm. Chem., 1962, 5:651; International Patent Publication WO 9854132). In hydrogenation (reduction) reactions that employ Raney Nickel, rhodium-alumina, palladium-carbon etc. as catalyst, dangerous and explosive hydrogen is used, which makes it difficult to obtain, selectively, the amidine derivatives of interest when high pressure is needed or when certain substrates are used.

On the other hand, biphenylamidine derivatives have been found as anti-coagulant agent having an excellent effect of inhibiting the activated blood coagulation factor X (hereinafter referred to as FXa) in International Patent Publication WO 99/26918, wherein intermediates biphenylamidine derivatives have been obtained by allowing an alcohol to react with the corresponding nitrile derivatives under an acidic condition in the presence of hydrogen chloride to prepare imidates, and then ammonia is allowed to react thereon (Pinner method). In this method, however, imidates, the reaction intermediates, are highly reactive, and, though ammonia is used in the conversion from imidates to amidine derivatives, the amidino groups formed are susceptible to basic condition, and the like, and thus care must be taken regarding the reaction condition such as temperature.

Thus, the above-mentioned methods of production have the following drawbacks:
1) The reduction reaction of amidoxime groups employs dangerous and explosive hydrogen gas, and high pressure is needed in some cases; and
2) Since the reaction proceeds via unstable reaction intermediates in the Pinner method, it is difficult to control the reaction.

Thus, these methods are far from satisfactory as methods for industrial production of amidine derivatives, and thus there has been a need for the development of methods that perform selective amidination under safe and mild conditions.

### Disclosure of the Invention

It is an object of the present invention to provide a method of preparing amidine derivatives in a simple and efficient manner under mild conditions using safe intermediates without using dangerous and explosive hydrogen gas in the reduction of amidoxime groups in the amidination reaction from nitrile derivatives via amidoximes.

Considering the above-mentioned conventional methods, intensive and extensive studies were made on the condition of amidoxime reduction, in methods of preparing amidine derivatives from nitrile derivatives via safe intermediates amidoximes, and the present inventors have discovered a novel method of preparing amidine derivatives without using hydrogen gas, as shown below.

Thus, the present invention provides a process for production of an amidine derivative represented by the following Formula (II): 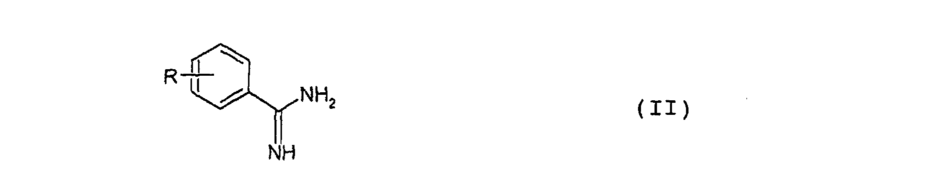 [wherein, R represents a hydrogen atom, an unsubstituted or substituted phenyl group, an unsubstituted or substituted C1-C10 alkyl group, an unsubstituted or substituted C1-C10 alkoxy group, an unsubstituted or substituted C1-C10 alkoxycarbonyl group, or a hydroxyl group present at the ortho, meta, or para position]
or a salt thereof,
comprising the steps of:
reducing an amidoxime derivative represented by the following Formula (I): 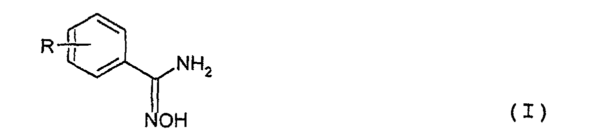 [wherein, R represents a hydrogen atom, an unsubstituted or substituted phenyl group, an unsubstituted or substituted C1-C10 alkyl group, an unsubstituted or substituted C1-C10 alkoxy group, an unsubstituted or substituted C1-C10 alkoxycarbonyl group, or a hydroxyl group present at the ortho, meta, or para position],
with zinc in an acetic acid as a solvent.

The above compound of Formula (I) can be obtained by reacting a nitrile derivative represented by the following Formula (III): 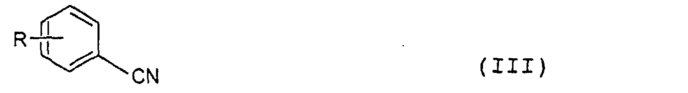 [wherein, R represents a hydrogen atom, an unsubstituted or substituted phenyl group, an unsubstituted or substituted C1-C10 alkyl group, an unsubstituted or substituted C1-C10 alkoxy group, an unsubstituted or substituted C1-C10 alkoxycarbonyl group, or a hydroxyl group present at the ortho, meta, or para position],
with hydroxylamine.

The present invention also provides an amidoxime derivative represented by the following Formula (V): 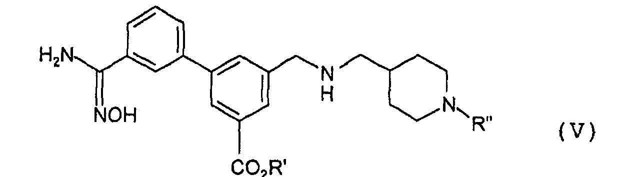 [wherein
R' represents a hydrogen atom, or an unsubstituted or substituted C1-C10 alkyl group, and
R" represents a hydrogen atom, or an unsubstituted or substituted C1-C10 alkoxycarbonyl group]
which is a novel and useful intermediate obtained by the above method of preparation,
or a salt thereof.

### Brief Explanation of the Drawings

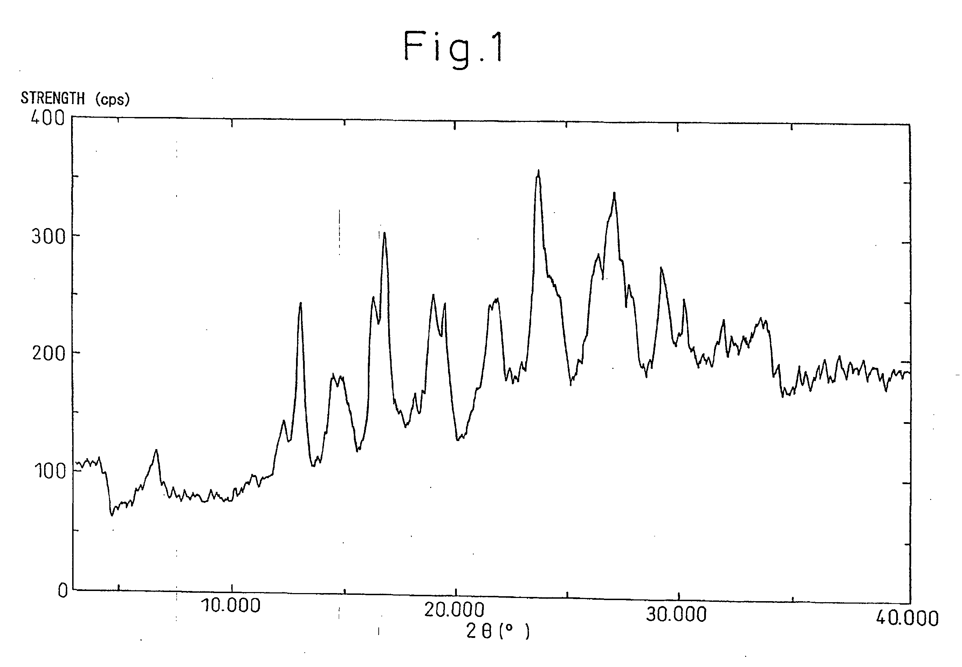

Fig. 1 shows an X-ray diffraction spectrum of methyl 3-(3-amidinopheny)-5-({[(4-piperidyl)methyl]amino}methyl)benzoate.1.5 zinc chloride.trihydrochloride.dihydrate.

### Best Mode for Carrying Out the Invention

The above amidoxime derivative (I) can be obtained from the reaction of a nitrile derivative represented by the following Formula (III): 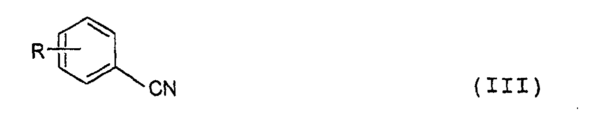 [wherein, R represents a hydrogen atom, an unsubstituted or substituted phenyl group, an unsubstituted or substituted C1-C10 alkyl group, an unsubstituted or substituted C1-C10 alkoxy group, an unsubstituted or substituted C1-C10 alkoxycarbonyl group, or a hydroxyl group present at the ortho, meta, or para position],
with hydroxylamine.

Solvents for use in the conversion of the nitrile derivative (III) to the amidoxime derivative (I) are not specifically limited as long as they do not affect the reaction, and include, for example, alcoholic solvents such as methanol, ethanol, propanol and isopropanol, and methanol and ethanol are preferred with methanol being most preferred. Bases for use in this reaction are not specifically limited as long as they do not affect the reaction, and include, for example, organic bases such as trimethylamine, triethylamine, tripropylamine, tributylamine, diisopropylethylamine, and DBU (1,8-diazabicyclo[5.4.0]-7-undecene) etc., and triethylamine is preferred.

The conversion of the amidoxime derivative (I) to the amidine derivative (II) is carried out by using zinc in an acetic acid as a solvent, whereas an excess amount of zinc is used in this reaction over the amidoxime, and is generally 2 to 100 equivalents, and preferably 2 to 10 equivalents. The reaction is generally carried out at a temperature of 40°C to 150°C in order to increase the reactivity of zinc, and is preferably carried out at 60°C to 100°C. The reaction time may vary depending on the reactivity of the amidoxime derivative (I), the amount of zinc used, and the reaction temperature, and is generally one hour to 24 hours, and preferably one hour to 12 hours.

The amidine derivative (II) thus obtained, after removing the excess amount of zinc by filtration, may be subjected to a purification procedure, if desired, to obtain a highly purified amidine derivative (II). The above series of reactions are novel and useful methods as a conversion of nitrile derivatives to amidine derivatives.

Furthermore, the present invention relates to a process for production of an amidine derivative represented by the following Formula (VI): 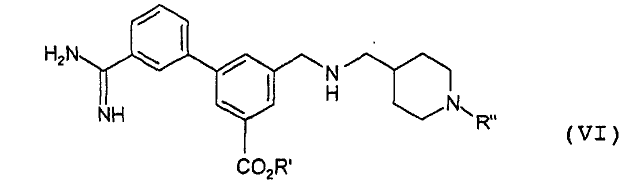 [wherein
R' represents a hydrogen atom, or an unsubstituted or substituted C1-C10 alkyl group, and
R" represents a hydrogen atom, or an unsubstituted or substituted C1-C10 alkoxycarbonyl group]
or a salt thereof,
comprising the steps of :
reacting a nitrile derivative represented by the following Formula (IV): 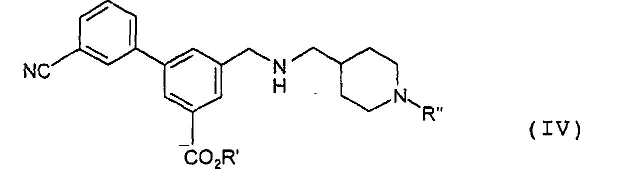 [wherein
R' represents a hydrogen atom, or an unsubstituted or substituted C1-C10 alkyl group, and
R" represents a hydrogen atom, or an unsubstituted or substituted C1-C10 alkoxycarbonyl group],
with hydroxylamine so as to give an amidoxime derivative represented by the following Formula (V): 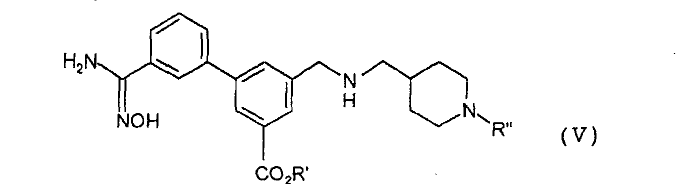
[wherein
R' represents a hydrogen atom, or an unsubstituted or substituted C1-C10 alkyl group, and
R" represents a hydrogen atom, or an unsubstituted or substituted C1-C10 alkoxycarbonyl group], and then
reducing the amidoxime group with zinc in an acetic acid as a solvent.

The present invention also provides crystals of methyl 3-(3-amidinophenyl)-5-({[(4-piperidyl)methyl]amino}methyl)benzoate hydrochloride having main peaks in an X-ray diffraction at diffraction angles 2θ(°): 12.3, 13.0, 14.5, 14.9, 16.3, 16.8, 19.0, 19.5, 21.9, 23.8, 24.7, 26.4, 27.2, 27.9, 29.3, 30.3, 32.0, and 33.9, which is a product obtained by the above method of preparation, and an important intermediate in the preparation of biphenylamidine derivatives described in International Patent Publication WO 99/26918.

The present invention also provides crystals of methyl 3-(3-amidinophenyl)-5-({[(4-piperidyl)methyl]amino}methyl)benzoate.1.5 zinc chloride.trihydrochloride.dihydrate represented by the following Formula (X): 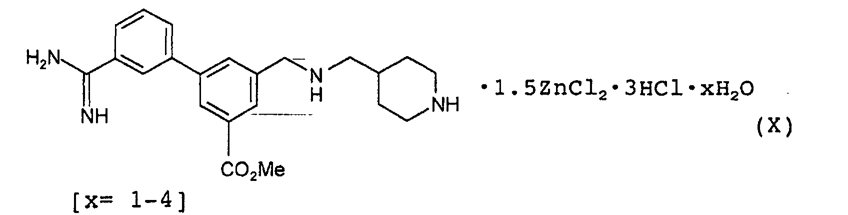 which is a product obtained by the above method of preparation, and an important intermediate in the preparation of biphenylamidine derivatives described in International Patent Publication WO 99/26918.

In the above definitions with regard to substituents of the compounds represented by the formulas (I) to (X) of the present invention, "substituted phenyl group" is not specifically limited as long as it does not affect the amidoxime-forming reaction or the amidination reaction, and includes, for example, an unsubstituted or substituted C1-C10 alkyl group, a carbonyl group, an unsubstituted or substituted C1-C10 alkoxycarbonyl group, an unsubstituted or substituted C1-C10 alkoxyl group, a hydroxyl group, and the like. Specifically, in the case of a substituted C1-C10 alkyl group as a substituent for the phenyl group, the [(N-unsubstituted or substituted piperidine-4-yl)methyl]aminomethyl group from the above Formula (IV) to (X) can be mentioned as a preferred example. Furthermore, in the case of a substituted C1-C10 alkoxycarbonyl group as a substituent for the phenyl group, the -CO2R' group from the above Formula (IV) to (X) can be mentioned as an example, and those in which it is methyl ester are specifically preferred.

The alkyl group in "unsubstituted or substituted C1-C10 alkyl group" means a chained (linear or branched) or circular alkyl group, and preferred examples thereof include a methyl group, an ethyl group, a propyl group, an isopropyl group, a butyl group, an iso-butyl group, a sec-butyl group, a tert-butyl group, a pentyl group, a neopentyl group, a 1-ethyl propyl group, a 2,2-dimethylpropyl group, a hexyl group, a 2-ethylbutyl group, a cyclopropyl group, a cyclopentyl group, a cyclohexyl group, and the like, and as most preferred examples there can be mentioned a methyl group and an ethyl group.

Substituents in "substituted C1-C10 alkyl group" are not specifically limited as long as long as they do not affect the amidoxime-forming reaction or the amidination reaction, and include, for example, a halogen atom, a hydroxyl group, an alkoxy group, and the like.

The C1-C10 alkoxylcarbonyl group in "unsubstituted or substituted C1-C10 alkoxycarbonyl group" means those having a chained (linear or branched) or circular alkyl group, and preferred examples thereof include a methoxycarbonyl group, an ethoxycarbonyl group, a propoxycarbonyl group, an isopropoxycarbonyl group, a butoxycarbonyl group, an isobutoxycarbonyl group, a sec-butoxycarbonyl group, a tert-butoxycarbonyl group, a pentyloxycarbonyl group, a hexyloxycarbonyl group and the like, and as most preferred examples there can be mentioned a tert-butoxycarbonyl group.

"Unsubstituted or substituted C1-C10 alkoxy group" means those having a chained (linear or branched) or circular alkyl group, and preferred examples thereof include a methoxy group, an ethoxy group, a propoxy group, an isopropoxy group, a butoxy group, an isobutoxy group, a sec-butoxy group, a tert-butoxy group, a pentyloxy group, a hexyloxy group, and the like.

### Examples

The present invention will now be explained more specifically with reference to specific examples. However, it is to be noted that the scope of the present invention is not limited by these examples in any way.

### Example 1. Synthesis of methyl 3-{3-[amino(hydroxyimino)methyl]phenyl}-5-({[(1-tert-butoxycarbonyl-4-piperidyl)methyl]amino}methyl)benzoate (the following Formula (XI))

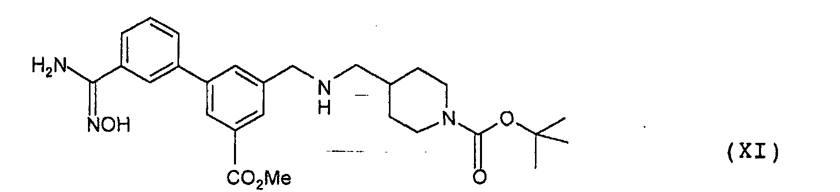

167.12 g of methyl 3-(3-cyanophenyl)-5-({[(1-tert-butoxycarbonyl-4-piperidyl)methyl]amino}methyl)benzoate (obtained by the method described in International Patent Publication WO 99/26918) was dissolved in 1.6 L of methanol, and 28.05 g of hydroxylamine hydrochloride and 56 ml of triethylamine were added thereto, and then stirred for 24 hours while heating the oil bath to 50°C. After completion of the reaction, solvents were evaporated from the reaction mixture under reduced pressure to give the title compound (the above Formula (XI)). The structure of the compound thus obtained was confirmed by mass spectrometric analysis.
[M+H]=497.

### Example 2. Methyl 3-(3-amidinophenyl)-5-({[(4-piperidyl)methyl]amino}methyl)benzoate·1.5 zinc chloride.trihydrochloride.dihydrate (the following Formula (XII))

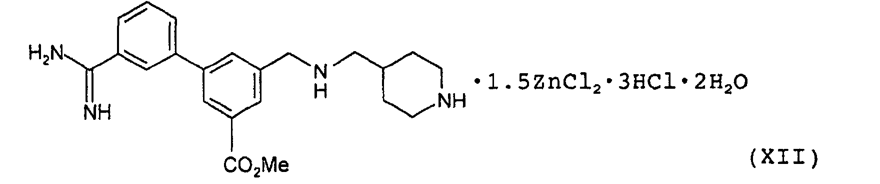

After 1.5 L of acetic acid was added to the compound obtained in Example 1, and the solution was rendered homogeneous, 87.98 g of zinc was added and stirred for 6 hours under heating at 80°C. After the residual zinc powder was removed by filtration, and the filtrate was concentrated under reduced pressure, 1640 ml of methanol was added to the residue to make a homogeneous solution. While stirring the solution, it was purged with hydrogen chloride gas for 40 minutes. The reaction mixture was stirred for 16 hours at room temperature. The crystals that deposited from the reaction solution were collected by filtration, and then dried at 50°C under reduced pressure to give 188.36 g of crude product of the title compound (the above Formula (XII)). Then, the crude product obtained was recrystallized using 570 ml of water and 1140 ml of 2-propanol to give 136.83 g of the title compound (yield from Example 1: 52%). The X-ray diffraction spectrum obtained is shown in Fig. 1.
1H-NMR (200 MHz, δ ppm, DMSO-d6+D₂O) 1.3-1.5 (m, 2H), 1.9-2.1 (m, 3H) 2.7-3.0 (m, 4H), 3.97 (s, 3H), 4.32 (s, 2H), 7.76 (t, J=7.8 Hz, 1H), 7.89 (d, J=7.8 Hz, 1H), 8.1-8.5 (m, 5H).
Composition (elemental analysis, ion chromatography analysis, plasma atomic emission spectrometry);
Calculated (C₂₂H₂₈N₄O₂.1.5.ZnCl₂.3HCl.2H₂O, wt%): Zn (13.4); Cl (29.1); C (36.2); H (4.8); N (7.7)
Found: Zn (13.1); Cl (29.5); C (35.7); H (4.5); N (7.5).

### Example 3. Methyl 3-{3-[amino(hydroxyimino)methyl]phenyl-5-({[(4-piperidyl)methyl]amino}methyl)benzoate (the following Formula (XIII))

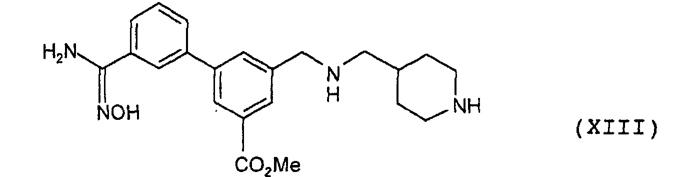

25.2 g of methyl 3-(3-cyanophenyl)-5-({[(4-piperidyl)methyl]amino}methyl)benzoate was dissolved in 250 ml of methanol, and 5.51 g of hydroxylamine hydrochloride and 5.1 ml of triethylamine were added thereto, and then stirred for 5 hours at 80°C. After completion of the reaction, solvents were evaporated from the reaction mixture under reduced pressure to give the title compound (the above Formula (XIII)). The structure of the compound thus obtained was confirmed by mass spectrometric analysis.
[M+H]=397.

### Example 4 . Methyl 3-(3-amidinophenyl)-5-({[(4-piperidyl)methyl]amino}methyl)benzoate·1.5 zinc chloride.trihydrochloride.dihydrate (the following Formula (XIV))

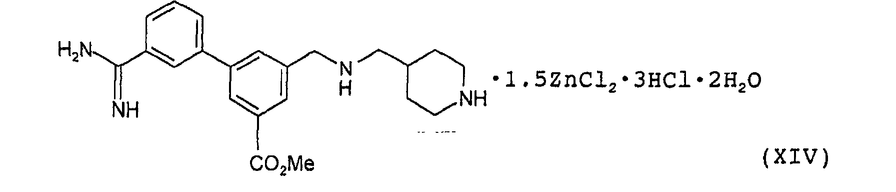

After 210 ml of acetic acid was added to the compound obtained in Example 3 and the solution was rendered homogeneous, 13.6 g of zinc was added thereto and stirred for 3 hours after heating to 80°C. After the residual zinc powder was removed by filtration, the filtrate was concentrated under reduced pressure. To the concentrate was added 210 ml of methanol to make a homogeneous solution, and then hydrogen chloride gas was purged thereinto for 40 minutes, and it was stirred at room temperature for 16 hours. The crystals that deposited were collected by filtration and then dried at 50°C under reduced pressure to give 31.92 g of the title compound (the above Formula (XIV)) (yield from Example 3: 69%).
1H-NMR (200 MHz, δ ppm, DMSO-d6+D₂O) 1.3-1.5 (m, 2H), 1.9-2.1 (m, 3H) 2.7-3.0 (m, 4H), 3.97 (s, 3H), 4.32 (s, 2H), 7.76 (t, J=7.8 Hz, 1H), 7.89 (d, J=7.8 Hz, 1H), 8.1-8.5 (m, 5H).
Composition (elemental analysis, ion chromatography analysis, plasma atomic emission spectrometry);
Calculated (C₂₂H₂₈N₄O₂.1.5ZnCl₂.3HCl.2H₂O, wt%): Zn (13.4); Cl (29.1); C (36.2); H (4.8); N (7.7)
Found: Zn (13.1); Cl (29.5); C (35.7); H (4.5); N (7.5).

### Industrial Applicability

According to the present invention, amidine derivatives can be produced via stable amidoxime intermediates derived from nitrile derivatives in a simple procedure and under a mild condition without using dangerous and explosive hydrogen gas for the reduction of amidoxime group. Furthermore, in the above method of production, there are provided crystals of methyl 3-(3-amidinophenyl)-5-({[(4-piperidyl)methyl]amino}methyl)benzoate hydrochloride that is a novel and useful intermediate for the production of anticoagulant biphenylamidine derivatives having an excellent effect of inhibiting FXa as described in International Patent Publication WO 99/26918.

## Claims

1. A process for production of an amidine derivative represented by the following Formula (II): 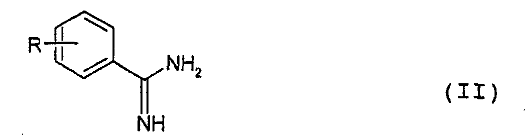 [wherein, R represents a hydrogen atom, an unsubstituted or substituted phenyl group, an unsubstituted or substituted C1-C10 alkyl group, an unsubstituted or substituted C1-C10 alkoxy group, an unsubstituted or substituted C1-C10 alkoxycarbonyl group, or a hydroxyl group present at the ortho, meta, or para position],
or a salt thereof,
comprising the steps of:
reducing an amidoxime derivative represented by the following Formula (I): 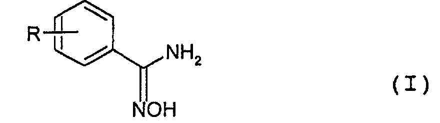 [wherein, R represents a hydrogen atom, an unsubstituted or substituted phenyl group, an unsubstituted or substituted C1-C10 alkyl group, an unsubstituted or substituted C1-C10 alkoxy group, an unsubstituted or substituted C1-C10 alkoxycarbonyl group, or a hydroxyl group present at the ortho, meta, or para position]
with zinc in an acetic acid as a solvent.

2. A process for production of an amidine derivative represented by the following Formula (II): 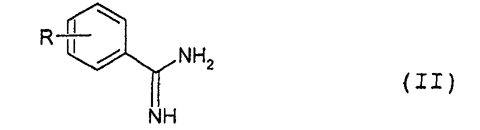 [wherein, R represents a hydrogen atom, an unsubstituted or substituted phenyl group, an unsubstituted or substituted C1-C10 alkyl group, an unsubstituted or substituted C1-C10 alkoxy group, an unsubstituted or substituted C1-C10 alkoxycarbonyl group or a hydroxyl group present at the ortho, meta, or para position],
or a salt thereof,
comprising the steps of:
reacting a nitrile derivative represented by the following Formula (III): 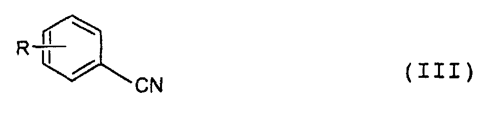 [wherein, R represents a hydrogen atom, an unsubstituted or substituted phenyl group, an unsubstituted or substituted C1-C10 alkyl group, an unsubstituted or substituted C1-C10 alkoxy group, an unsubstituted or substituted C1-C10 alkoxycarbonyl group, or a hydroxyl group present at the ortho, meta, or para position]
with hydroxylamine so as to give an amidoxime derivative represented by the following Formula (I): 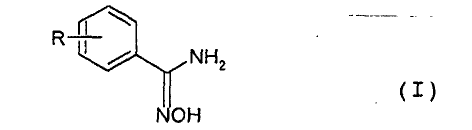 [wherein, R represents a hydrogen atom, an unsubstituted or substituted phenyl group, an unsubstituted or substituted C1-C10 alkyl group, an unsubstituted or substituted C1-C10 alkoxy group, an unsubstituted or substituted C1-C10 alkoxycarbonyl group, or a hydroxyl group present at the ortho, meta, or para position], and then
reducing the amidoxime group with zinc in an acetic acid as a solvent.

3. A process for production of an amidine derivative represented by the following Formula (VI): 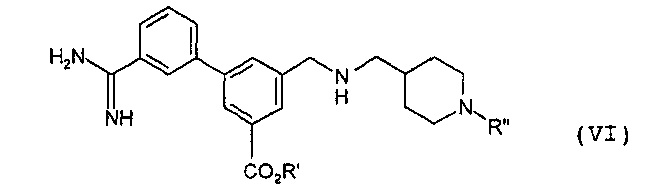 [wherein
R' represents a hydrogen atom, or an unsubstituted or substituted C1-C10 alkyl group, and
R" represents a hydrogen atom, or an unsubstituted or substituted C1-C10 alkoxycarbonyl group],
or a salt thereof,
comprising the steps of:
reacting a nitrile derivative represented by the following Formula (IV): 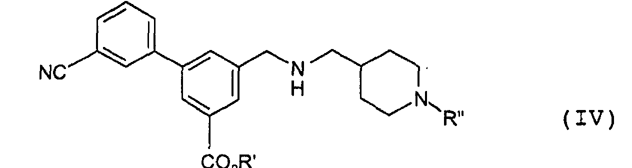
[wherein,
R' represents a hydrogen atom, or an unsubstituted or substituted C1-C10 alkyl group, and
R" represents a hydrogen atom, or an-unsubstituted or substituted C1-C10 alkoxycarbonyl group]
with hydroxylamine so as to give an amidoxime derivative represented by the following Formula (V): 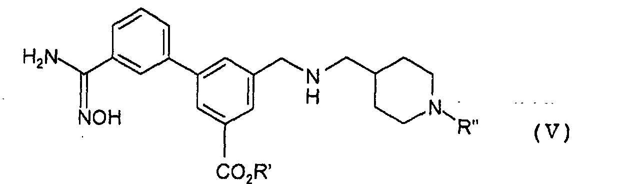
[wherein,
R' represents a hydrogen atom, or an unsubstituted or substituted C1-C10 alkyl group, and
R" represents a hydrogen atom, or an unsubstituted or substituted C1-C10 alkoxycarbonyl group], and then
reducing the amidoxime group with zinc in an acetic acid as a solvent.

4. A process for production of an amidine derivative represented by the following Formula (IX): 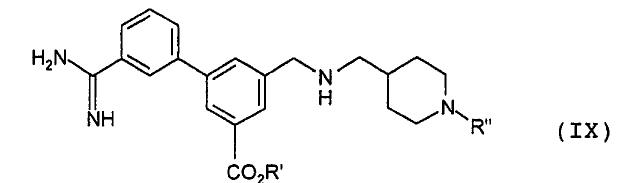 [wherein
R' represents an unsubstituted or substituted C1-C10 alkyl group, and
R" represents a tert-butoxycarbonyl group]
and the salt thereof,
comprising the steps of:
reacting a nitrile derivative represented by the following Formula (VII): 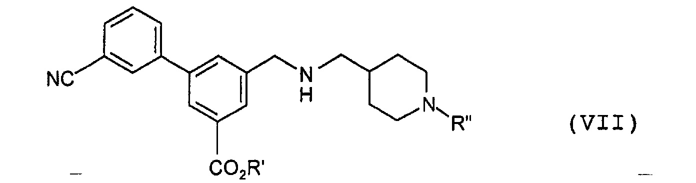
[wherein,
R' represents an unsubstituted or substituted C1-C10 alkyl group, and
R" represents a tert-butoxycarbonyl group]
with hydroxylamine so as to give an amidoxime derivative represented by the following Formula (VIII): 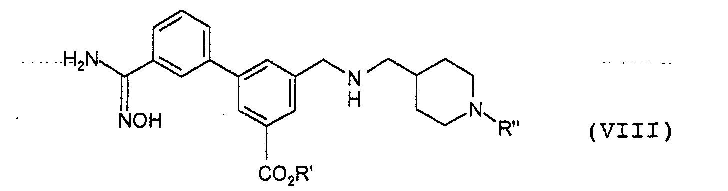
[wherein,
R' represents an unsubstituted or substituted C1-C10 alkyl group, and
R" represents a tert-butoxycarbonyl group], and then
reducing the amidoxime group with zinc in an acetic acid as a solvent.

5. An amidoxime derivative represented by the following Formula (V): 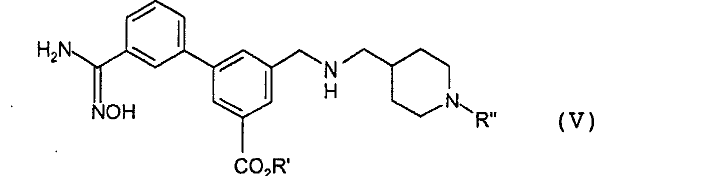 [wherein,
R' represents a hydrogen atom, or an unsubstituted or substituted C1-C10 alkyl group, and
R" represents a hydrogen atom, or an unsubstituted or substituted C1-C10 alkoxycarbonyl group],
or salt thereof.

6. Crystals of methyl 3-(3-amidinophenyl)-5-({[(4-piperidyl)methyl]amino}methyl)benzoate hydrochloride having main peaks in an X-ray diffraction at diffraction angles 2θ(°): 12.3, 13.0, 14.5, 14.9, 16.3, 16.8, 19.0, 19.5, 21.9, 23.8, 24.7, 26.4, 27.2, 27.9, 29.3, 30.3, 32.0, and 33.9.

7. Crystals of methyl 3-(3-amidinophenyl)-5-({[(4-piperidyl)methyl]amino}methyl)benzoate·1.5 zinc chloride.trihydrochloride.hydrate represented by the following Formula (X):
